# EUROPÄISCHE PATENTSCHRIFT

(11) **EP 0 657 448 B1**
(45) Veröffentlichungstag und Bekanntmachung des Hinweises auf die Patenterteilung: **04.03.1998**
(21) Anmeldenummer: 94118676.9
(22) Anmeldetag: 28.11.1994
(51) Int. Cl.: C07D 401/04, C07D 471/04

(54) **Eintopfverfahren zur Herstellung von 3-Chinoloncarbonsäurederivaten**
One pot process for the preparation of 3-quinolonecarboxylic acid derivatives
Procédé à un seul réacteur pour la préparation des dérivés de l'acide 3-quinolonecarboxylique

(30) Priorität: 10.12.1993 DE 4342186
(43) Veröffentlichungstag der Anmeldung: 14.06.1995
(73) Patentinhaber: BAYER AG, 51368 Leverkusen (DE)
(72) Erfinder: Zerbes, Rudolf Dr., D-42113 Wuppertal (DE); Naab, Paul Dr., D-42287 Wuppertal (DE); Franckowiak, Gerhard Dr., D-42115 Wuppertal (DE); Diehl, Herbert Dr., D-51373 Leverkusen (DE)

(56) Entgegenhaltungen:
- EP-A- 0 167 763
- EP-A- 0 300 311
- EP-A- 0 550 903

## Beschreibung

Die vorliegende Erfindung betrifft ein Eintopfverfahren zur Herstellung von 7-Heterocyclyl-substituierten 3-Chinoloncarbonsäurederivaten. Derartige Verbindungen sind an sich bekannt. Sie haben eine starke antimikrobielle Wirkung. Zu ihnen gehören Wirkstoffe wie z.B. Ofloxacin, Ciprofloxacin oder Enrofloxacin.

Erfindungsgemäß herzustellende Verbindungen aus dieser Klasse sind in 7-Position mit Heterocyclen substituiert, die als Heteroatom zumindest ein Stickstoffatom enthalten aber zusätzlich auch Sauerstoff, Schwefel oder weitere Stickstoffatome enthalten können. Diese Heterocyclen können auch substituiert sein. Als Beispiele seien für monocyclische Substituenten Piperazinyl, N-Ethylpiperazinyl, Pyrrolidinyl, 3-Aminopyrrolidinyl, Morpholinyl oder Thiomorpholinyl genannt.

In einer Ausführungsform betrifft die vorliegende Erfindung solche 3-Chinoloncarbonsäurederivate, die in 7-Position mit einem bicyclischen Heterocyclus substituiert sind, also ein Eintopfverfahren zur Herstellung von 3-Chinoloncarbonsäurederivaten der allgemeinen Formel 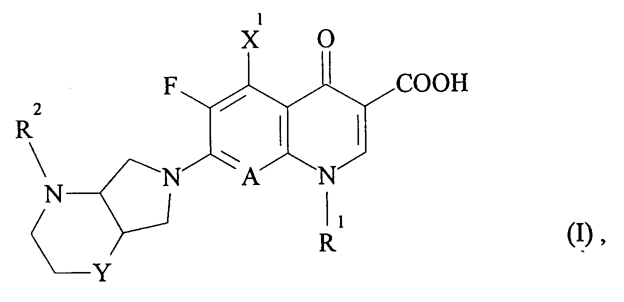 in welcher
- A: für CH, CF, CCl, C-OCH₃, C-CH₃,
- X¹: für H, Halogen, NH₂, CH₃,
- Y: für CH₂ oder O,
- R¹: für C₁-C₃-Alkyl, FCH₂CH₂-, Cyclopropyl, gegebenenfalls durch Halogen ein-bis dreifach substituiertes Phenyl oder Cyclopropyl steht,
- R²: für Wasserstoff, 5-Methyl-2-oxo-1,3-dioxolen-4-yl-methyl, C₂-C₅-Oxoalkyl, CH₂-CO-C₆H₅, CH₂CH₂CO₂R⁶, R⁶O₂C-CH=C-CO₂R⁶, -CH=CH-CO₂R⁶ oder CH₂CH₂-CN,
worin
- R⁶: Wasserstoff oder C₁-C₃-Alkyl bedeutet,
steht.

Derartige Chinoloncarbonsäurederivate sind wertvolle pharmazeutische Wirkstoffe. Sie sind zur Herstellung von antimikrobiellen Mitteln geeignet.

Bisher sind verschiedene Verfahren zur Herstellung von Chinoloncarbonsäurederivaten bekannt geworden.

Nach EP-A-0 167 763 werden Verbindungen der Formel (II) (Z¹,Z²,Z³ = unabhängig voneinander Fluor oder Chlor) 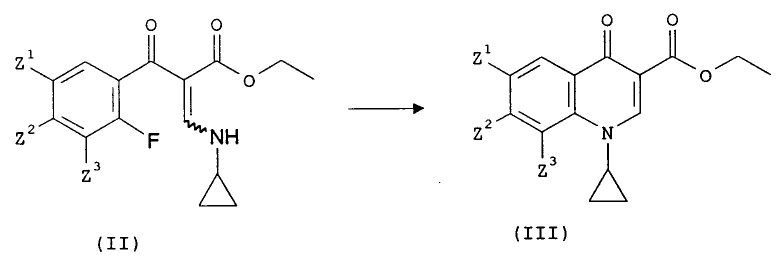 bei Temperaturen von 60°C bis 300°C in Gegenwart einer Base wie Alkalifluorid oder -carbonat in einem Lösungsmittel wie DMF, HMPT oder NMP cyclisiert. Der Ester (III) wird in einem nächsten Schritt zur Säure (IV) hydrolysiert. Diese wird anschließend mit gegebenenfalls cyclischen Aminen (V) vorzugsweise in Lösungsmitteln zu den substituierten Derivaten (VI) umgesetzt.

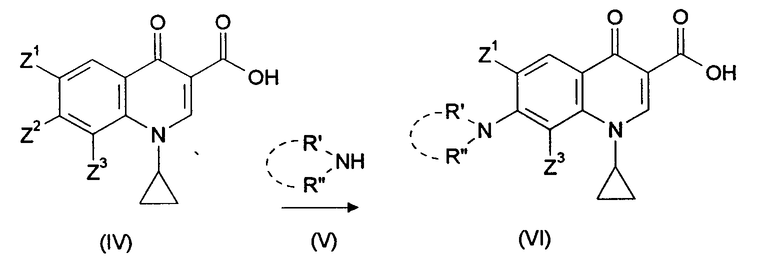

Die Gesamtausbeute von (II) zu (VI) ist mit 56 % jedoch unbefriedigend. Zudem können bei der alkalischen Verseifung des Esters (III) Nebenprodukte auftreten, in denen Z² durch Hydroxy oder Alkoxy substituiert wurde, sowie Oligo- und Polymere, insbesondere wenn Z² für Fluor steht. Führt man die Verseifung unter sauren Bedingungen durch, wird Fluorwasserstoff freigesetzt, was zur Korrosion der Produktionsanlage und zur Verunreinigung des Produktes mit komplexen Metallfluoriden führt. Das gilt insbesondere wenn nach der Cyclisierung von (II) zu (III), bei der HF entsteht und an eine Base gebunden wird, diese Reaktionsmischung ohne vorherige Isolierung des Esters (III) zur sauren Hydrolyse eingesetzt wird, wobei zwangsläufig der zuvor an Base gebundene Fluorwasserstoff wieder freigesetzt wird.

Gemäß EP-A-0 275 971 können Verbindungen der Formel (XI) erhalten werden, indem das Amin (V') bereits vor dem Ringschluß eingeführt wird:

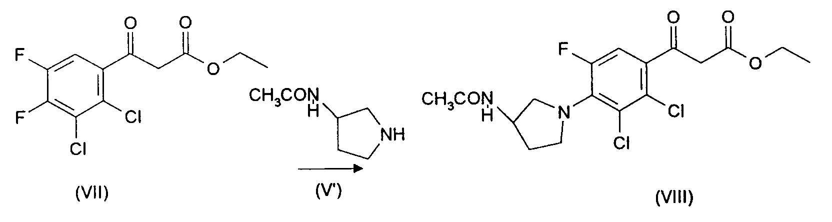

Anschließend wird die der vorstehenden Verbindung (II) entsprechende Cyclopropylaminoacrylat-Vorstufe (IX) synthetisiert:

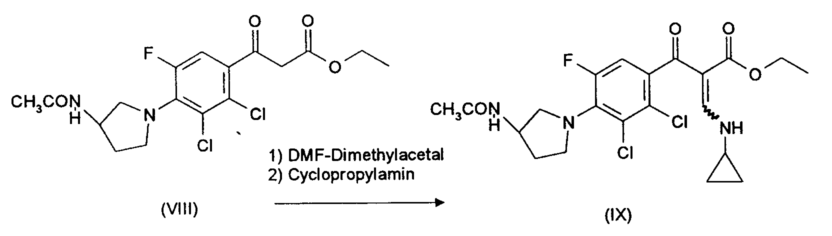

Die Cyclisierung von (IX) in Gegenwart von starken Basen wie z.B. NaH führt zum Ester (X):

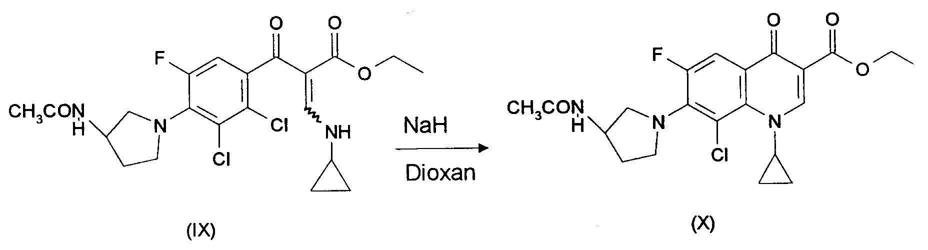

Durch Hydrolyse von (X) wird schließlich die Zielverbindung (XI) erhalten:

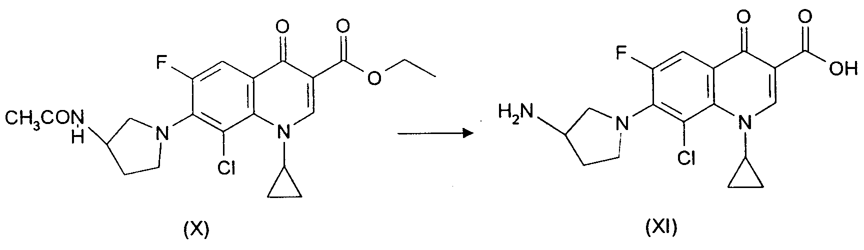

Die Gesamtausbeute von (VII) zu (XI) beträgt jedoch nur 15 %, so daß ein derartiges Verfahren sehr unwirtschaftlich ist.

Auch aus der EP-A-0 350 733 ist ein mehrstufiges Verfahren bekannt, nach welchem antibakteriell wirksame Chinoloncarbonsäuren durch Umsetzung der Carbonsäuren (XII, R³ = H) mit zum Teil bicyclischen Aminen erhalten werden.

Die Ausbeute ist, ausgehend von der Aminoacrylatvorstufe, mit ca. 60% jedoch nicht zufriedenstellend. Zudem existiert auch hier das Problem der unerwünschten Substitution des 7-Halogens durch Hydroxy oder Alkoxy, speziell bei der alkalischen Hydrolyse des Esters (XII, R³ = Alkyl). 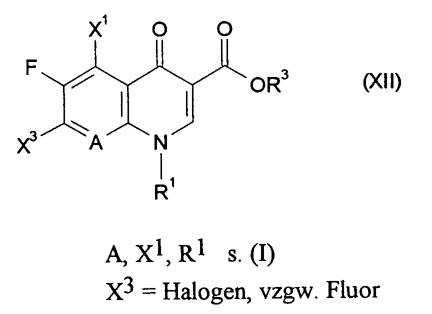
A, X¹, R¹ s. (I)
X³ = Halogen, vzgw. Fluor

Diese Nachteile - mehrstufige Synthese, niedrige Ausbeuten, Bildung korrosiver Spaltprodukte, Verunreinigung der Endprodukte - erschweren die großtechnische Produktion dieser Wirkstoffe.

Zur Vermeidung vielstufiger Syntheseverfahren kann die Entwicklung von sogenannten Eintopfverfahren erwogen werden. Hierbei wird die Synthese ohne Isolation der Zwischenstufen in demselben Reaktionsgefäß durch aufeinander folgende Zugabe der Reaktanden durchgeführt. Ein derartiges Verfahren zur Synthese von Vorstufen des Typs (IV) von Chinoloncarbonsäurederivaten (VI) ist aus der EP-A-0 300 311 bekannt.

Das dort beschriebene Verfahren endet jedoch auf der Stufe einer Chinoloncarbonsäure (analog IV), die anschließend mit dem in 7-Position einzuführenden Amin umgesetzt werden müßte. Die vorstehend beschriebenen Nachteile können demnach nicht vermieden werden.

Es wurde nun ein vorteilhaftes Eintopfverfahren zur Herstellung von 7-heterocyclylsubstituierten Chinoloncarbonsäurederivaten gefunden, bei welchem nicht nur die vorstehend beschriebenen Nachteile hinsichtlich der Bildung von unerwünschten 7-Hydroxy-Nebenprodukten und Anlagenkorrosion durch freigesetzten Fluorwasserstoff absolut entfallen. Die gewünschten Wirkstoffe (I) sind zudem in hoher Reinheit mit Ausbeuten von meist über 90 %, bezogen auf die Stufe (XIII) oder (XXVI) zugänglich.

Erfindungsgemäß wird ein Säurehalogenid der Formel (XXVI) 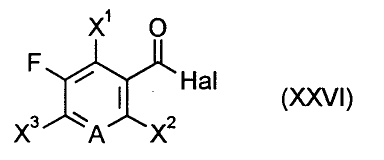 in welcher
Hal, X² und X³ für Fluor oder Chlor stehen und
A und X¹ die für Formel (I) angegebene Bedeutung haben,
entweder mit einem Dimethylaminoacrylsäureester der Formel (CH₃)₂N-CH=CH-COOR in einem Lösungsmittel umgesetzt, wonach durch Zugabe eines Amins R¹-NH₂ unter Aminaustausch im Acrylesterteil des Primärproduktes der Aminoacrylester (XIII) erzeugt wird oder das Säurehalogenid (XXVI) wird gleich mit einem Aminoacrylester der Formel R¹NH-CH=CH-COOR umgesetzt, in welchem Fall der vorstehend beschriebene Aminaustausch entfällt und sogleich die Verbindung (XIII) erzeugt wird.

Dieser Aminoacrylester der Formel (XIII), in welcher A, X¹, R¹ die für Formel (I) angegebene Bedeutung haben, X² und X³ für Halogen und R für einen üblichen, zur Esterbildung geeigneten organischen Rest, vorzugsweise Methyl, Ethyl oder Propyl, steht, wird im selben Lösungsmittel mit einer Hilfsbase erwärmt und dabei zum Ester (XIV) cyclisiert.

Anschließend wird der Heterocyclus, z.B. das Amin (XVII) zu dieser Mischung gegeben und nach Abschluß der Bildung des 7-Substitutionsproduktes (XVI) die 3-Esterfunktion durch die Zugabe einer starken Base verseift. Die Reaktionsmischung wird abschließend mit einer Säure neutral gestellt, und das ausfallende Produkt (I) wird isoliert. Das folgende Formelschema erläutert beispielhaft die Reaktionsfolge dieses Eintopfverfahrens:

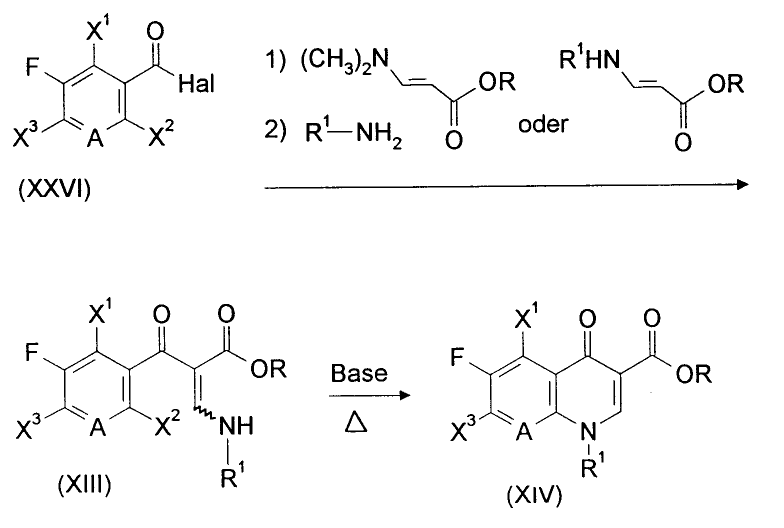

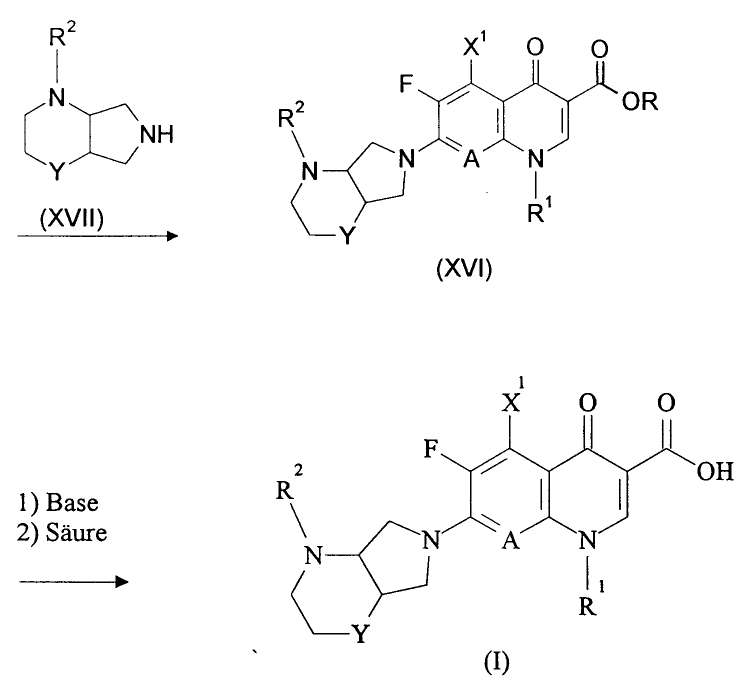

In den Formeln (I) sowie (XIII), (XIV), (XVI) und (XVII) haben die Symbole im allgemeinen die folgende Bedeutung:
- A: steht für CH, CF, CCl, C-OCH₃, C-CH₃,
- R: steht für einen üblichen, zur Esterbildung geeigneten organischen Rest, vorzugsweise Ethyl, Methyl oder Propyl,
- R¹: steht für C₁-C₃-Alkyl, FCH₂CH₂-, Cyclopropyl, gegebenenfalls durch Halogen ein- bis dreifach substituiertes Phenyl oder Cyclopropyl,
- R²: steht für Wasserstoff, 5-Methyl-2-oxo-1,3-dioxolen-4-yl-methyl, C₂-C₅-Oxoalkyl, CH₂-CO-C₆H₅, CH₂CH₂CO₂R⁶, R'O₂C-CH=C-CO₂R⁶, -CH=CH-CO₂R⁶ oder CH₂CH₂-CN,
worin
- R⁶: Wasserstoff oder C₁-C₃-Alkyl bedeutet,
- Y: steht für CH₂ oder O,
- X¹: steht für H, Halogen, NH₂, CH₃ und
- X² und X³: stehen für Halogen.

Bevorzugt wird erfindungsgemäß die Synthese von Verbindungen (I) mit Vorstufen (XIII) und (XVII) durchgeführt in denen
- A: für CH, CF, CCl, C-OCH₃, C-CH₃,
- R: für C₁-C₄-Alkyl, C₆H₅, Si(CH₃)₃,
- R¹: für C₂H₅, gegebenenfalls ein- bis dreifach durch Fluor substituiertes Cyclopropyl oder 2,4-Difluorphenyl,
- R²: für H, CH₂-O-CH₃, CH₂-O-C₆H₅, CH₂CH₂-CO-CH₃, CH₂CH₂CO₂R⁶, R⁶O₂C-CH=C-CO₂R⁶, -CH=CO₂R⁶, CH₂CH₂CN, worin R⁶ C₁-C₃-Alkyl bedeutet,
- Y: für CH₂ oder O,
- X¹: für Wasserstoff oder Halogen steht und
- X² und X³: für Chlor oder Fluor stehen.

Besonders bevorzugt sind solche Verbindungen der obengenannten Formeln, in denen
- A: für CCl oder CF,
- R: für CH₃ oder C₂H₅,
- R¹: für Cyclopropyl,
- R²: für Wasserstoff,
- Y: für CH₂
- X¹: für Wasserstoff,
- X²: für Fluor oder Chlor und
- X³: für Fluor steht.

Erfindungsgemäß hergestellt werden auch 3-Chinoloncarbonsäurederivate der allgemeinen Formel (Ib), 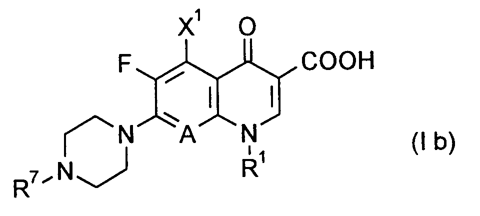 in welcher
- A: für CH, CF, CCl, C-OCH₃, C-CH₃,
- X¹: für H, Halogen, NH₂, CH₃,
- R¹: für C₁-C₃-Alkyl, FCH₂CH₂-, Cyclopropyl, gegebenenfalls durch Halogen ein-bis dreifach substituiertes Phenyl oder Cyclopropyl und
- R⁷: für Wasserstoff, gegebenenfalls substituiertes Alkyl oder Phenyl oder eine übliche zum Schutz eines Stickstoffatoms geeigneten Gruppe, vorzugsweise aber Ethyl steht,
indem ohne eine Isolation der Zwischenstufen ein Säurehalogenid der Formel (XXVI) 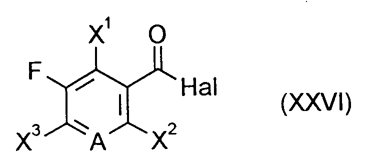 in welcher
Hal, X² und X³ für Fluor oder Chlor stehen und A und X¹ die vorstehende Bedeutung haben, mit einem Dimethylaminoacrylsäureester der Formel

(CH₃)₂N-CH=CH-COOR

in einem Lösungsmittel umgesetzt wird, wonach durch Zugabe eines Amins R¹-NH₂ unter Aminaustausch im Acrylesterteil des Primärproduktes der Aminoacrylester (XIII) erzeugt wird, 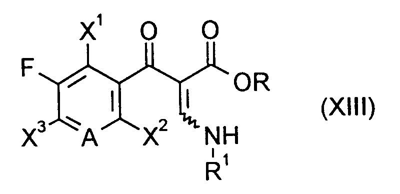 in welcher A, X¹ und R¹ die für die Formel (I b) angegebene Bedeutung haben, X² und X³ für Halogen und R für einen üblichen zur Esterbildung geeigneten organischen Rest, vorzugsweise Methyl, Ethyl, Propyl, steht, in einem Lösungsmittel mit einer Hilfsbase erwärmt und dabei zu Verbindungen der allgemeinen Formel (XIV) cyclisiert werden, 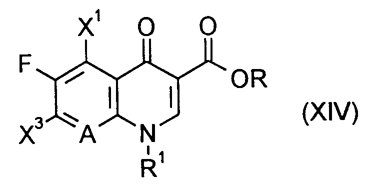 in welcher A, R, R¹, X¹ und X³ die vorgenannte Bedeutung haben,
welche durch Umsetzung mit Verbindungen der allgemeinen Formel (XV) 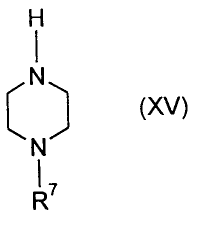 in welcher
- R⁷: die vorgenannte Bedeutung hat,
in Ester der allgemeinen Formel (XXVII) 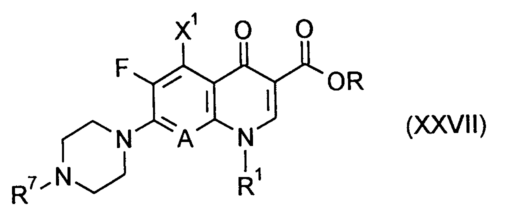 in welcher A, R, R¹, R⁷ und X¹ die vorgenannte Bedeutung haben, überführt werden, aus welchen durch alkalische Verseifüng der Esterfunktion die 3-Chinoloncarbonsäurederivate der Formel (I b) entstehen, die durch Neutralisation des Reaktionsgemisches ausgefällt werden.

Zu den besonders bevorzugt mit dem erfindungsgemäßen Verfahren herzustellenden Verbindungen (I) gehören auch optisch aktive Derivate, erhältlich durch Umsetzung von (XIII) mit enantiomerenreinen Aminen (XVII a-d), die in DE-A-4 208 789 und DE-A-4 208 792 beschrieben sind.

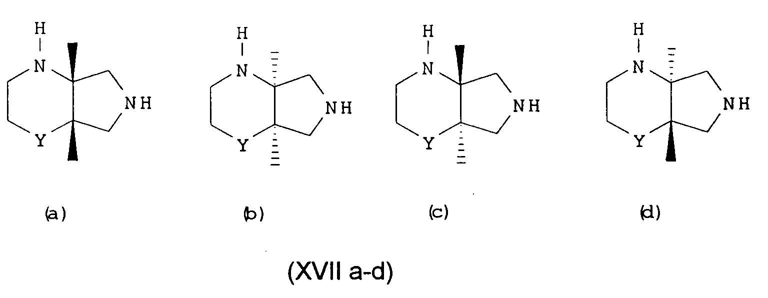

Besonders bevorzugte erfindungsgemäß herstellbare Einzelverbindungen sind z.B. die

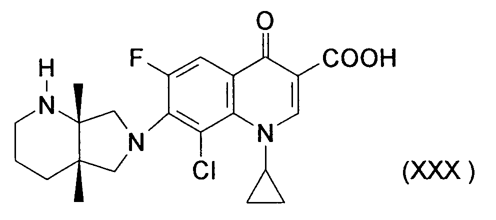

8-Chlor-1-cyclopropyl-7([S,S]-2,8-diazabicyclo[4.3.0]non-8-yl)-6-fluor-1,4-dihydro-4-oxo-3-chinolincarbonsäure (XXX) und die

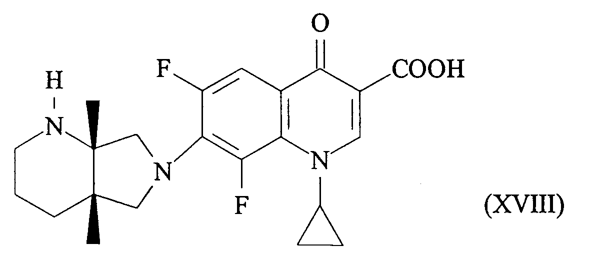

1-Cyclopropyl-7([S,S]-2,8-diazabicyclo[4.3.0]non-8-yl)-6,8-difluor-1,4-dihydro-4-oxo-3-chinolincarbonsäure (XVIII).

Zur Durchführung des erfindungsgemäßen Eintopfverfahrens können prinzipiell alle gängigen inerten organischen Lösungsmittel verwendet werden. Als Beispiele seien Dimethylethylenharnstoff (DMEU), Dimethylpropylenharnstoff (DMPU), N-Methylcaprolactam, tert.-Butanol, Tetramethylharnstoff, Sulfolan und Dimethoxyethan genannt. Vorzugsweise werden mit Wasser mischbare Lösungsmittel wie N-Methylpyrrolidon (NMP), Diglyme, N,N-Dimethylformamid (DMF) oder Dioxan verwendet, besonders bevorzugt NMP.

Die Cyclisierung wird bei niedrigstmöglicher Temperatur durchgeführt. Allgemein sind Temperaturen von 60°C bis 100°C ausreichend, was an Hand orientierender Vorversuche mit der ausgewählten Vorstufe (XIII) im ausgewählten Medium Lösungsmittel/Hilfsbase ebenso leicht ermittelt werden kann wie die optimale Lösungsmittelmenge.

Als Hilfsbasen können bei der Cyclisierung in der organischen Synthese übliche Säurebinder verwendet werden. Beispielhaft können für diese Reaktionsstufe Kalium-tert.-butanolat, Butyl-lithium, Lithium-phenyl, Natriummethylat, Natriumhydrid, Natrium- oder Kaliumcarbonat und Kalium-oder Natriumfluorid genannt werden. Es kann vorteilhaft sein, einen Überschuß von bis zu 10 Mol-% an Base einzusetzen, gegebenenfalls auch höher.

Bevorzugt werden Natrium- oder Kaliumcarbonat eingesetzt.

Nach Beendigung der Cyclisierung wird bei gleicher, gegebenenfalls weiter erhöhter Temperatur z.B. das Amin (XV) oder (XVII) zudosiert. Die optimale Reaktionstemperatur ist von den Reaktionspartnern abhängig, kann jedoch wiederum ohne weiteres in einem Vorversuch ermittelt werden. Im allgemeinen sind Temperaturen von 60°C bis 100°C ausreichend.

Nach Abschluß der Substitution der 7-Position in (XIV) wird das Reaktionsgemisch durch Zugabe von Wasser verdünnt und abgekühlt. Allgemein wird man etwa das Volumen des Reaktionsgemisches an Wasser verwenden. Zur Verseifung der Esterfünktion wird dann Alkalilauge zugegeben, vorzugsweise Natronlauge, in äquimolarer Menge oder bis zu einem Überschuß von etwa 10 Mol-%. Die Verseifüng wird vorzugsweise bei ca. 60°C durchgeführt.

Anschließend wird das Reaktionsgemisch weiter mit Wasser verdünnt, wobei man in der Regel etwa das doppelte Volumen des Gemisches an Wasser zugeben wird. Mit Mineralsäure, vorzugsweise Salzsäure, oder Essigsäure, wird der pH-Wert auf ca. 7,8 eingestellt und gegebenenfalls weiter auf 0 bis 5°C abgekühlt.

Das hierbei ausfallende Zielprodukt z.B. (I) oder (Ib) wird abschließend z.B. durch Abnutschen isoliert.

In der Regel erhält man das Produkt z.B. (I) oder (Ib) in einer Reinheit von >95 % in einer Ausbeute von >85 %, meist jedoch >90 % bezogen auf die Ausgangsverbindung (XIII).

Die folgenden Ausführungsbeispiele erläutern die Erfindung:
1.) Synthese von 8-Chlor-1-cyclopropyl-([S,S]-2,8-diazabicyclo[4.3.0]non-8-yl)-6-fluor-1,4-dihydro-4-oxo-3-chinolincarbonsäure (XXX): 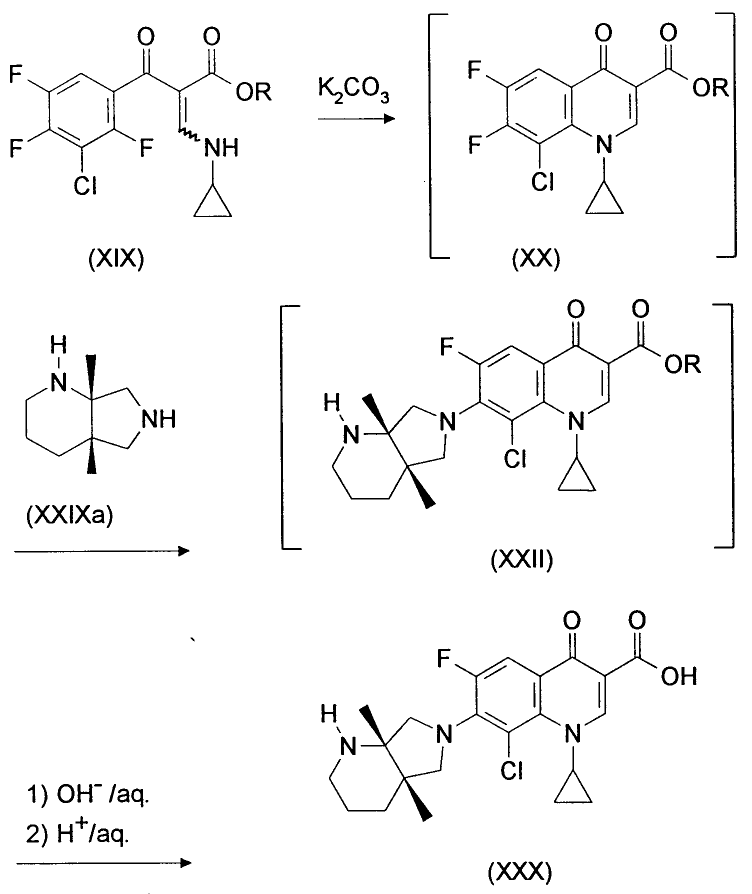
   a) Via Methylester (R = CH₃)
      10,2 g K₂CO₃ und 17,7g (0,053 m) XIX (Methylester) werden in 30 ml NMP 50 min auf 60°C erhitzt.
      7,5 g (0,059 m) S,S-Pyrrolopiperidin (XXIXa) werden zugegeben und 90 min bei 90°C gerührt.
      Der entstandene Ester (XXII) wird mit 33 g 8,5 %iger Natronlauge in 50 min bei 60°C verseift. Nach Verdünnen des Ansatzes mit 120 ml Wasser wird mit 6n Salzsäure auf pH 7.8 eingestellt.
      Nach Abkühlen auf 5°C trennt man das ausgefallene Produkt (XXX) auf einer Nutsche ab, wäscht mit 3 x 100 ml Wasser nach und trocknet bei 80°C im Vakuum über Nacht.
      - Ausbeute:: 19 g ≙ 86,1 % d. Th. (bei 97,5 Gew.-%)
   b) Via Ethylester (R = C₂H₅)
      20,4g K₂CO₃ und 36,8g (0,106 m) (XIX) werden in 60 ml Diglyme 2,5 Stunden auf 80°C erhitzt.
      Nach Zugabe von 15 g (0,118 m) S,S-Pyrrolopiperidin (XXIXa) wird 4 Stunden bei 90 bis 100°C gerührt.
      Der entstandene Ester (XXII) wird nach Zugabe von 60 ml Wasser und 22 g 45 %iger Natronlauge in 2,5 Stunden bei 80°C verseift. Der Ansatz wird mit 240 ml Wasser verdünnt und mit 6n-Salzsäure auf pH 7.8 eingestellt.
      Nach Abkühlen auf 5°C wird das Produkt (XXX) abgesaugt, mit Wasser gewaschen und bei 70°C im Vakuum getrocknet.
      - Ausbeute:: 43 g ≙ 96,5 % d. Th. (bei 96,5 Gew.-%)
2) Synthese von 1-Cyclopropyl-7([S,S]-2,8-diazabicyclo[4.3.0]non-8-yl)-6,8-difluor-1,4-dihydro-4-oxo-3-chinolincarbonsäure (XVIII). 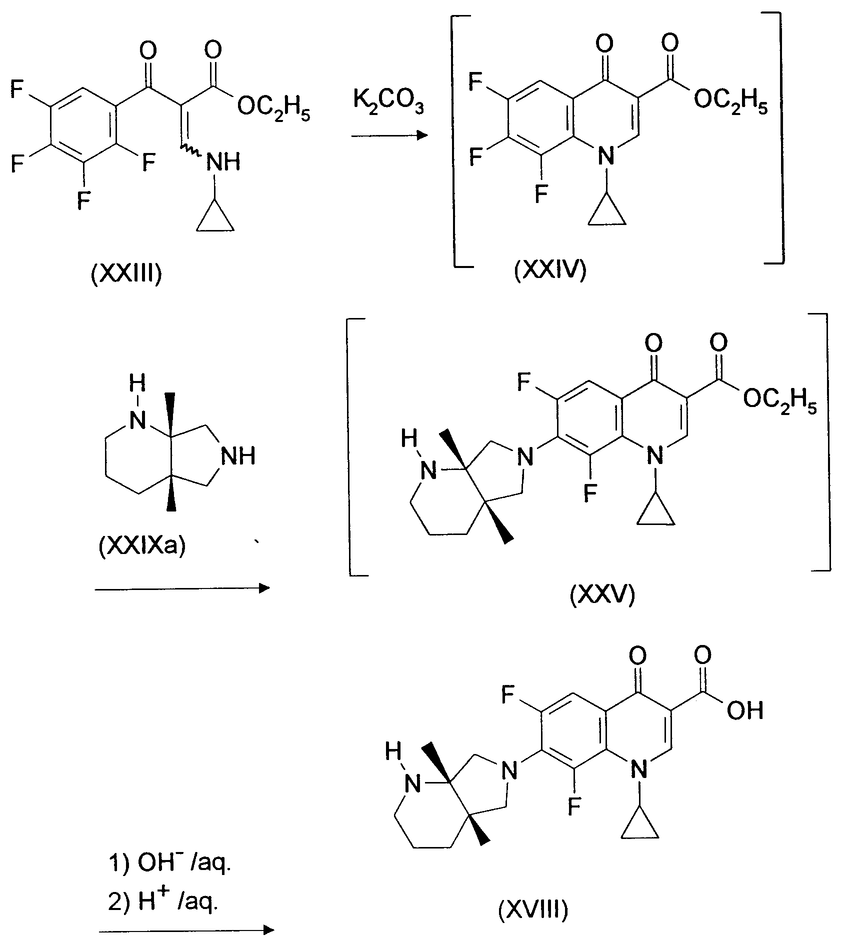
   20,4g K₂CO₃ und 35,2g (0,106 m) (XXIII) werden in 60 ml NMP 1 Stunde auf 60°C erwärmt. (Nach 40 min sind nach HPLC 97,6 % (XXIV) entstanden).
   Man gibt 15,5 g (0,12 m) S,S-Pyrrolopiperidin (XXIXa) zu. Nach 2 Stunden 80°C sind noch 0,2 % (XXIV) vorhanden (HPLC).
   Nach Zugabe von 60 ml Wasser und 12 g NaOH wird der entstandene Ester (XXV) in 4 Stunden/60°C zu (XVIII) verseift.
   Man verdünnt mit 240 ml Wasser und stellt mit 6n Satzsaure auf pH 7,8 ein.
   Das Produkt wird abgesaugt, mit Wasser gewaschen und im Vakuum bei 70°C getrocknet.
   - Ausbeute:: 38,1 ≙ 91,4 % d. Th. (bei 98,9 Gew.-%)
3. Synthese von 1-Cyclopropyl-6-fluor-7-(1-piperazinyl)- und -7-(4-ethyl-1-piperazinyl)-1,4-dihydro-4-oxo-3-chinolincarbonsäure (XXXIVa, b) 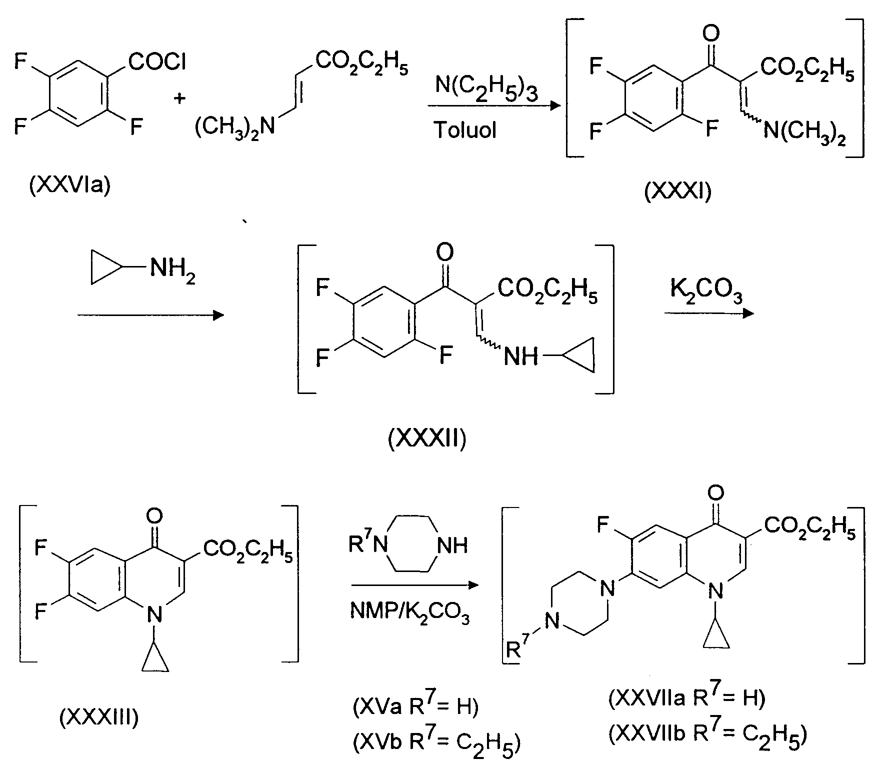 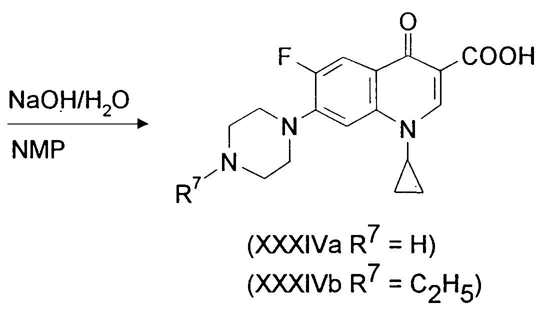
   35,8 g (0,25 m) Dimethylaminoacrylsäureethylester und 27,3 g (0,27 m) Triethylamin werden in 50 ml Toluol vorgelegt und bei 50°C in 30 Minuten 48,6 g (0,25 m) 2,4,5-Trifluorbenzoylchlorid (XXVIa) zugetropft. Es wird 1 Stunde nachgerührt bei 50 bis 55°C und anschließend 17,3 g (0,28 m) Eisessig und 15,5 g (0,27 m) Cyclopropylamin bei 30 bis 36°C zugetropft. Nach 1 Stunde werden die Salze mit 100 ml Wasser extrahiert. Die organische Phase wird im Wasserstrahl-Vakuum bei 40°C eingedampft. Man erhält 80,3 g (XXXII) als öligen Rückstand.
   Durch Zugabe von 250 ml N-Methylpyrrolidon wird das Öl (XXXII) gelöst und mit 48,4 g (0,35 m) Pottasche auf 80 bis 90°C erwärmt. Nach 1 Stunde werden 86 g (1 m) Piperazin (XVa) bzw. 114g (1 m) N-Ethylpiperazin (XVb) zugegeben. Man rührt eine Stunde bei 80 bis 90°C und verdünnt den Ansatz mit 150 ml Wasser.
   Nach Zugabe von 20 g (0,5 m) NaOH hält man die Temperatur 1 Stunde bei 70°C und verdünnt anschließend weiter mit 500 ml Wasser. Geringe Verunreinigungen der Lösung werden abfiltiert und der pH-Wert mit halbkonzentrierter Salzsäure auf 7,5 eingestellt.
   Nach Abkühlen auf 0 bis 5°C wird das Betain (XXXIV) nach 2 Stunden abgesaugt, mit 2 x 200 ml Wasser gewaschen und das Produkt über Nacht im Vakuum getrocknet.
   - Ausbeuten:: XXXIVa 71,4g (98,0 Gew.-%, HPLC) ≙ 84,5 % der Theorie
   XXXIVb 78,2 g (98,5 Gew.-%, HPLC) ≙ 87,0 % der Theorie
4) Synthese von 1-Cyclopropyl-6-fluor-7(1-piperazinyl)- und -7-(4-ethyl-1-piperazinyl)-1,4-dihydro-4-oxo-3-chinolincarbonsäure (XXXIVa, XXXIVb) 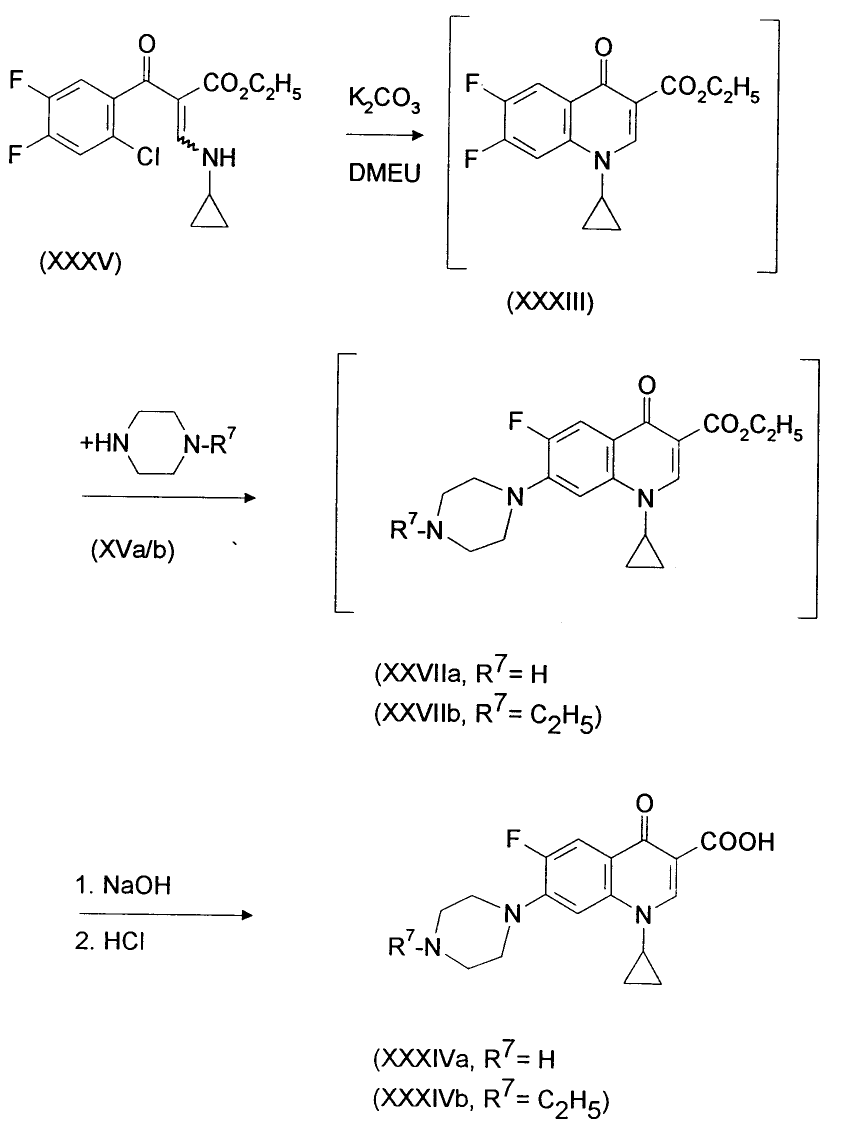
   82,4 g (XXXV) wird in 250 ml DMEU gelöst und mit 48,4 g (0,35 m) Pottasche auf 100 bis 120°C erwärmt.
   Nach 2 Stunden werden 86 g (1 m) Piperazin (XVa) bzw. 114 g (1m) N-Ethylpiperazin (XVb) zugegeben. Man rührt eine Stunde bei 80 bis 90°C und verdünnt den Ansatz mit 150 ml Wasser.
   Nach Zugabe von 20 g (0,5 m) NaOH hält man die Temperatur 1 Stunde bei 70°C und verdünnt anschließend weiter mit 500 ml Wasser. Geringe Verunreinigungen der Lösung werden abfiltriert und der pH-Wert mit hälbkonzentrierter Salzsäure auf 7,5 eingestellt.
   Nach Abkühlen auf 0 bis 5°C wird das Betain (XXXIV) nach 2 Stunden abgesaugt, mit 2 x 200 ml Wasser gewaschen und das Produkt über Nacht im Vakuum getrocknet.
   - Ausbeute: (XXXIVa): 73,0 g (98,5 Gew.-%) ≙ 86,8 % d. Th.
   (XXXIVb): 77,2g (99,0 Gew.-%) ≙ 85,1 % d. Th.
5. 7-(3-Amino-1-pyrrolidinyl)-8-chlor-1-cyclopropyl-6-fluor-1,4-dihydro-4-oxo-3-chinolincarbonsäure (XI) 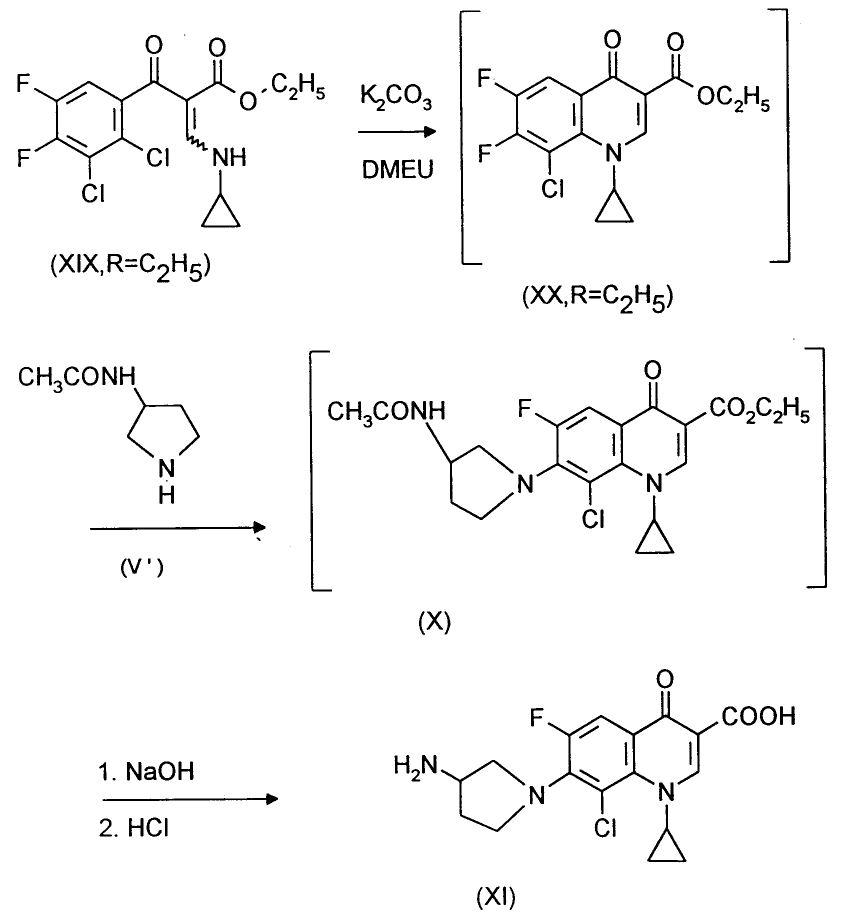
   20,6 g (XIX, R = C₂H₅) werden in 65 ml DMEU gelöst und mit 12,2 g K₂CO₃ 2 Stunden auf 100 bis 120°C erwärmt.
   Man gibt 8,5 g 3-Acetamidopyrrolidin (V') zu und rührt 1 Stunde bei 80°C bis 90°C nach. Anschließend verdünnt man die Reaktionsmischung mit 40 ml Wasser und gibt 10 g NaOH zu. Nach 4 Stunden bei 90 bis 100°C wird mit weiteren 125 ml Wasser verdünnt, filtriert und mit halbkonzentrierter Salzsäure neutralisiert.
   Der Feststoff wird abfiltriert, mit Wasser und Isopropanol gewaschen und über Nacht im Vakuum getrocknet.
   - Ausbeute:: 17,3 g (XI) (98,7 Gew.-% HPLC) ≙ 83 % d. Th.

### Beispiel

Synthese von 1-Cyclopropyl-6-fluor-7-(1-piperazinyl)-1,4-dihydro-4-oxo-3-chinolincarbonsäure (XXXIVa).

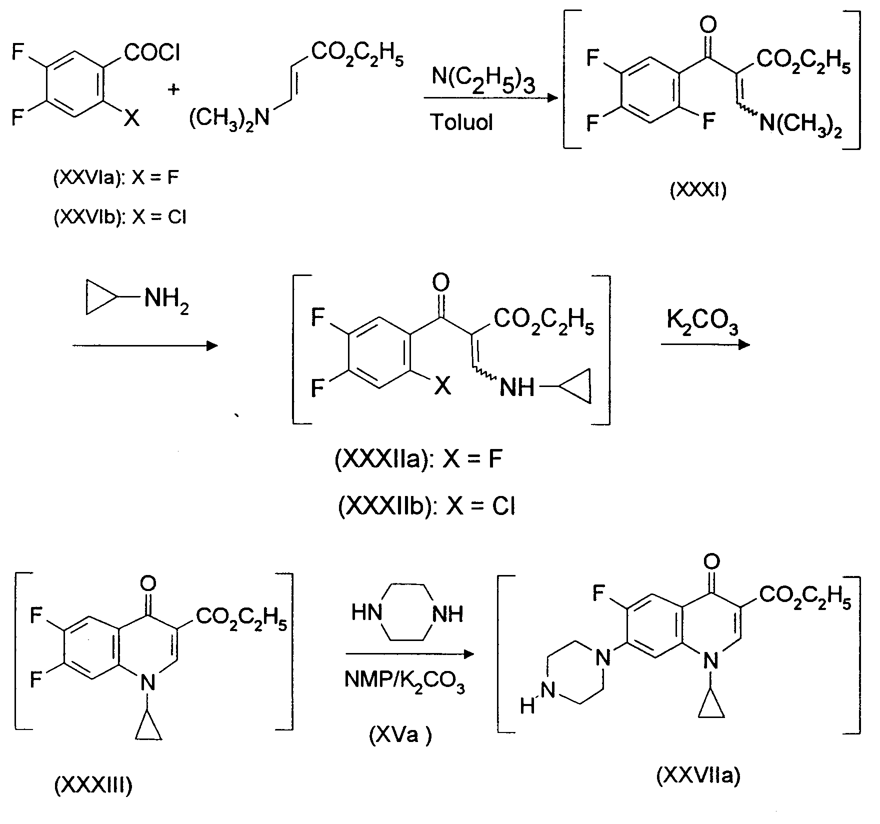

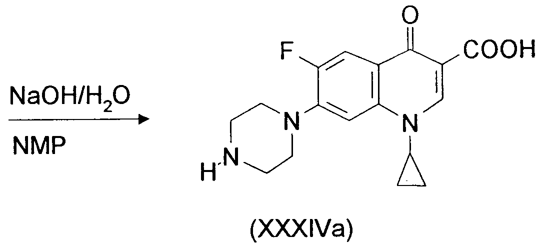

35,8 g (0.25 Mol) Dimethylaminoacrylsäureethylester und 27.3 g (0.27 Mol) Triethylamin werden in 50 ml Toluol vorgelegt und bei 50°C in 30 Minuten eine Mischung aus 24,3 g (0,125 Mol) 2,4-5-Trifluorbenzoylchorid (XXVIa) und 26,3 g (0,125 m) 2-Chlor-4,5-difluorbenzoylchlorid (XXVIb) zugetropft. Es wird 1 Stunde bei 50 bis 55°C nachgerührt und anschließend 17,3 g (0,28 Mol) Eisessig und 15,5 g (0,27 Mol) Cyclopropylamin bei 30 bis 36°C zugetropft. Nach 1 Stunde werden die Salze mit 100 ml Wasser extrahiert. Die organische Phase wird im Wasserstrahl-Vakuum bei 40°C eingedampft. Man erhält 81,5 g (XXXIIa/b) als öligen Rückstand.

Durch Zugabe von 250 ml N-Methylpyrrolidon wird das Öl (XXXIIa/b) gelöst und mit 48,4 g (0,35 Mol) Pottasche auf 90 bis 120°C erwärmt. Nach 2 Stunden werden 86 g (1 Mol) Piperazin (XVa) zugegeben. Man rührt eine Stunde bei 80 bis 90°C und verdünnt den Ansatz mit 150 ml Wasser.

Nach Zugabe von 20 g (0,5 Mol) NaOH hält man die Temperatur 1 Stunde bei 70°C und verdünnt anschließend weiter mit 500 ml Wasser. Geringe Verunreinigungen der Lösung werden abfiltriert und der pH-Wert mit halbkonzentrierter Salzsäure auf 7,5 eingestellt.

Nach Abkühlen auf 0 bis 5°C wird das Betain (XXXIV) nach 2 Stunden abgesaugt, mit 2 x 200 ml Wasser gewaschen und das Produkt über Nacht im Vakuum getrocknet.
- Ausbeute:: XXXIVa 73,4 (98,0 Gew.-%, HPLC) ≙ 86,5% der Theorie

## Patentansprüche

1. Eintopfverfahren zur Herstellung von 3-Chinoloncarbonsäurederivaten der allgemeinen Formel (Ia) 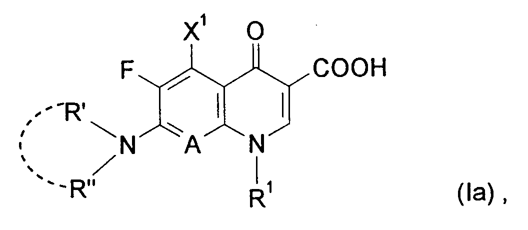 in der
R' und R'' zusammen mit dem Stickstoffatom, an das sie gebunden sind, einen mono- oder bicyclischen Heterocyclus bilden, der gegebenenfalls in allen Ringteilen weitere Stickstoff-, Sauerstoff- oder Schwefel-Heteroatome enthalten kann und der gegebenenfalls substituiert sein kann und
in welcher
A für CH, CF, CCl, C-OCH₃, C-CH₃,
X¹ für H, Halogen, NH₂, CH₃,
R¹ für C₁-C₃-Alkyl, FCH₂CH₂-, Cyclopropyl, gegebenenfalls durch Halogen ein- bis dreifach substituiertes Phenyl oder Cyclopropyl steht,
dadurch gekennzeichnet, daß ohne eine Isolation der Zwischenstufen Säurehalogenide der Formel (XXVI) 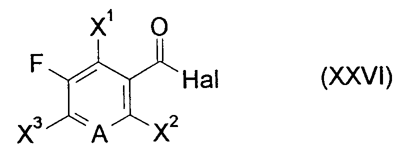 in welcher
Hal, X² und X³ für Fluor oder Chlor stehen und A und X¹ die oben angegebene Bedeutung haben, entweder mit einem Dimethylaminoacrylsäureester der Formel (CH₃)₂N-CH=CH-COOR in einem Lösungsmittel umgesetzt wird, wonach durch Zugabe eines Amins R¹-NH₂ unter Aminaustausch im Acrylesterteil des Primärproduktes der Aminoacrylester (XIII) erzeugt wird oder das Säurehalogenid gleich mit einem Aminoacrylester der Formel R¹NH-CH=CH-COOR umgesetzt wird, in welchem Fall der vorstehend beschriebene Aminaustausch entfällt und sogleich die Verbindung (XIII) erzeugt wird, 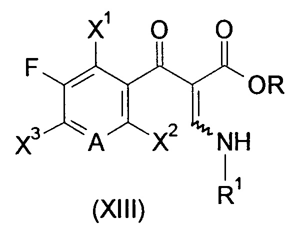 in welcher A, X¹ und R¹ die für Formel (Ia) angegebene Bedeutung haben, X² und X³ für Halogen und R für einen üblichen, zur Esterbildung geeigneten Rest, vorzugsweise Methyl, Ethyl oder Propyl steht, in einem Lösungsmittel mit einer Hilfsbase erwärmt und dabei zu Verbindungen der allgemeinen Formel (XIV) cyclisiert werden, 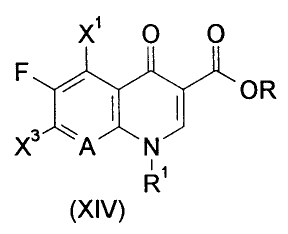 in welcher A, R, R¹ und X³ die vorgenannte Bedeutung haben,
welche ohne Zwischenisolierung durch Umsetzung mit heterocyclischen Verbindungen 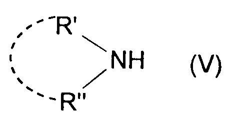 in welcher
R' und R'' die obengenannte Bedeutung haben
in Ester der allgemeinen Formel 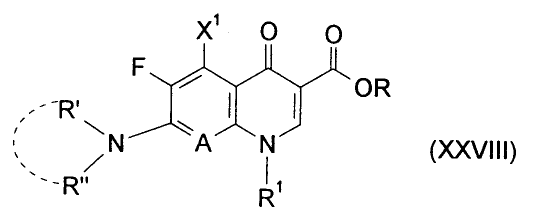 in welcher A, R, R¹, R', R'' und X¹ die vorgenannte Bedeutung haben, überführt werden, aus welchen durch alkalische Verseifung der Esterfunktion die 3-Chinoloncarbonsäurederivate der Formel (Ia) entstehen, die durch Neutralisation des Reaktionsgemisches ausgefällt werden.

2. Eintopfverfahren gemäß Anspruch 1, zur Herstellung von 3-Chinoloncarbonsäurederivaten der allgemeinen Formel (Ib), 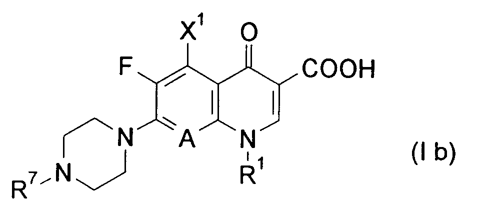 in welcher
A für CH, CF, CCl, C-OCH₃, C-CH₃,
X¹ für H, Halogen, NH₂, CH₃
R¹ für C₁-C₃-Alkyl, FCH₂CH₂-, Cyclopropyl, gegebenenfalls durch Halogen ein- bis dreifach substituiertes Phenyl oder Cyclopropyl und
R⁷ für Wasserstoff gegebenenfalls substituiertes Alkyl oder Phenyl oder eine übliche zum Schutz eines Stickstoffatoms geeigneten Gruppe, vorzugsweise aber Ethyl steht,
daduch gekennzeichnet, daß ohne eine Isolation der Zwischenstufen ein Säurehalogenid der Formel (XXVI) 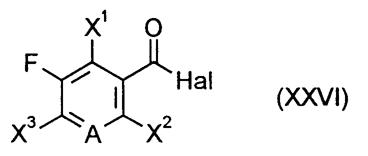 in welcher
Hal, X² und X³ für Fluor oder Chlor stehen und A und X¹ die Bedeutung gemäß Anspruch 1 haben, mit einem Dimethylaminoacrylsäureester der Formel
(CH₃)₂N-CH=CH-COOR
in einem Lösungsmittel umgesetzt wird, wonach durch Zugabe eines Amins R¹-NH₂ unter Aminaustausch im Acrylesterteil des Primärproduktes der Aminoacrylester (XIII) erzeugt wird, 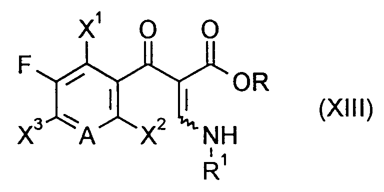 in welcher A, X¹ und R¹ die für die Formel (I b) angegebene Bedeutung haben, X² und X³ für Halogen und R für einen üblichen zur Esterbildung geeigneten organischen Rest, vorzugsweise Methyl, Ethyl, Propyl, steht, in einem Lösungsmittel mit einer Hilfsbase erwärmt und dabei zu Verbindungen der allgemeinen Formel (XIV) cyclisiert werden, 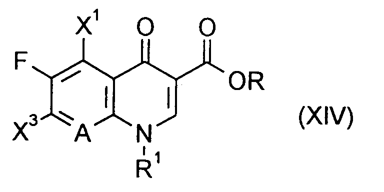 in welcher A, R, R¹, X¹ und X³ die vorgenannte Bedeutung haben, welche durch Umsetzung mit Verbindungen der allgemeinen Formel (XV) 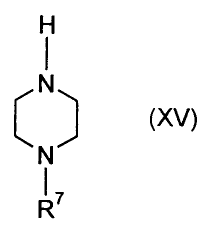 in welcher
R⁷ die vorgenannte Bedeutung hat,
in Ester der allgemeinen Formel (XXVII) 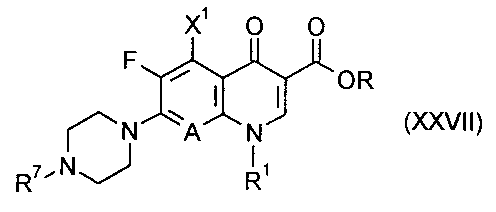 in welcher A, R, R¹, R⁷ und X¹ die vorgenannte Bedeutung haben, überführt werden, aus welchen durch alkalische Verseifüng der Esterfunktion die 3-Chinoloncarbonsäurederivate der Formel (I b) entstehen, die durch Neutralisation des Reaktionsgemisches ausgefällt werden.

3. Eintopfverfahren gemäß Anspruch 1 zur Herstellung von 3-Chinoloncarbonsäurederivaten der allgemeinen Formel (I) 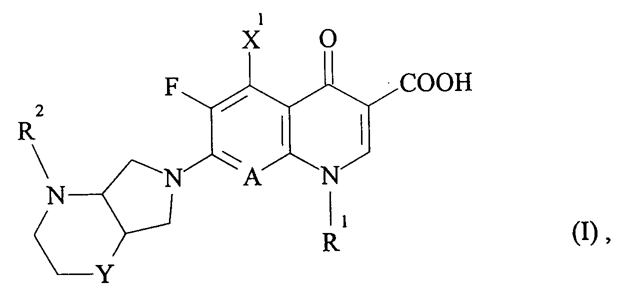 in welcher
A für CH, CF, CCl, C-OCH₃, C-CH₃,
X¹ für H, Halogen, NH₂, CH₃,
Y für CH₂ oder O,
R¹ für C₁-C₃-Alkyl, FCH₂CH₂-, Cyclopropyl, gegebenenfalls durch Halogen ein- bis dreifach substituiertes Phenyl oder Cyclopropyl steht,
R² für Wasserstoff 5-Methyl-2-oxo-1,3-dioxolen-4-yl-methyl, C₂-C₅-Oxoalkyl, CH₂-CO-C₆H₅, CH₂CH₂CO₂R⁶, R⁶O₂C-CH=C-CO₂R⁶, -CH=CH-CO₂R⁶ oder CH₂CH₂-CN steht,
worin
R⁶ Wasserstoff oder C₁-C₃-Alkyl bedeutet,
dadurch gekennzeichnet, daß ohne eine Isolation der Zwischenstufen ein Säurehalogenid der Formel 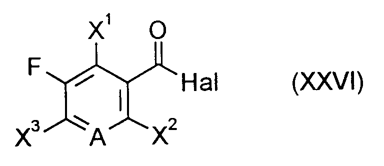 in welcher
Hal, X² und X³ für Fluor oder Chlor stehen und A und X¹ die für Formel (I) angegebene Bedeutung haben, mit einem Dimethylaminoacrylsäureester der Formel (CH₃)₂N-CH=CH-COOR in einem Lösungsmittel umgesetzt wird, wonach durch Zugabe eines Amins R¹-NH₂ unter Aminaustausch im Acrylesterteil des Primärproduktes der Aminoacrylester (XIII) erzeugt wird, 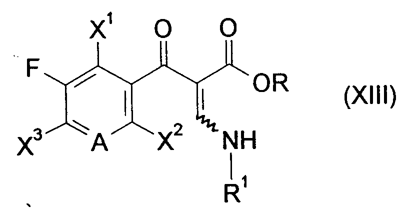 in welcher A, X¹ und R¹ die für Formel (I) angegebene Bedeutung haben, X² und X³ für Halogen und R für einen üblichen, zur Esterbildung geeigneten organischen Rest, vorzugsweise Methyl, Ethyl oder Phenyl steht, in einem Lösungsmittel mit einer Hilfsbase erwärmt und dabei zu Verbindungen der allgemeinen Formel (XIV) cyclisiert werden, 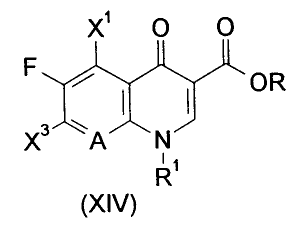 in welcher A, R, R¹, X¹ und X³ die vorgenannte Bedeutung haben,
welche durch Umsetzung mit Verbindungen der allgemeinen Formel (XVII) 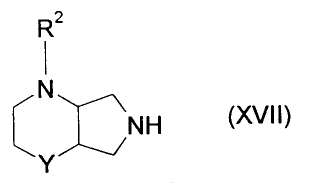 in welcher
R² für Wasserstoff, 5-Methyl-2-oxo-1,3-dioxolen-4-yl-methyl, C₂-C₅-Oxoalkyl, CH₂-CO-C₆H₅, CH₂CH₂CO₂R⁶, R⁶O₂C-CH=C-CO₂R⁶, -CH=CH-CO₂R⁶ oder CH₂CH₂-CN,
Y für CH₂ oder O steht,
in Ester der allgemeinen Formel (XVI) 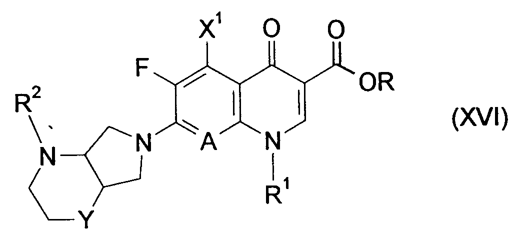 in welcher A, R, R¹, R², Y und X¹ die vorgenannte Bedeutung haben, überführt werden, aus welchen durch alkalische Verseitung der Esterfunktion die 3-Chinoloncarbonsäurederivate der Formel (I) entstehen, die durch Neutralisation des Reaktionsgemisches ausgefällt werden.

4. Verfahren gemäß Anspruch 3, dadurch gekennzeichnet, daß Acrylester der Formel (XIII) eingesetzt werden, in welchen
A für CH, CF, CCl, C-OCH₃, C-CH₃,
R für einen üblichen, zur Esterbildung geeigneten organischen Rest,
R¹ für C₁-C₃-Alkyl, FCH₂CH₂-, Cyclopropyl, gegebenenfalls durch Halogen ein- bis dreifach substituiertes Phenyl oder Cyclopropyl steht,
X¹ für H, Halogen, NH₂, CH₃ und
X² und X³ für Halogen stehen
und als Verbindungen der Formel (XVII) solche, in welchen
R² für H, CH₂-O-CH₃, CH₂-O-C₆H₅, CH₂CH₂-CO-CH₃, CH₂CH₂CO₂R⁶, R⁶O₂C-CH=C-CO₂R⁶, -CH=CO₂R⁶, CH₂CH₂CN steht,
worin
R⁶ C₁-C₃-Alkyl bedeutet und
Y für CH₂ oder O steht.

5. Verfahren gemäß Anspruch 2, dadurch gekennzeichnet, daß als Säurehalogenide der Formel (XXVI) solche eingesetzt werden, in welchen
A für CH,
X¹ für Wasserstoff,
X² für Fluor oder Chlor und
X³ für Fluor steht,
und als Verbindungen der Formel (XV) solche, in welchen
R⁷ für Wasserstoff oder Ethyl steht.

6. Verfahren gemäß Anspruch 4, dadurch gekennzeichnet, daß Acrylester der Formel (XIII) eingesetzt werden, in welchen
A für CCl oder CF
R für CH₃ oder C₂H₅
R¹ für Cyclopropyl
X¹ für Wasserstoff
X² für Fluor oder Chlor und
X³ für Fluor steht
und als Verbindungen (XVII) solche, in welchen
R² für Wasserstoff und
Y für CH₂ steht.

7. Verfahren gemäß Ansprüchen 1, 3, 4 und 6, dadurch gekennzeichnet, daß es sich bei den Verbindungen der Formel (XVII) um enantiomerenreine Stoffe handelt.

8. Verfahren gemäß Anspruch 6, dadurch gekennzeichnet, daß es sich bei der Verbindung der Formel (XVII) um eine enantiomerenreine Verbindung aus der Gruppe der Verbindungen der Formeln (XXIXa) bis (XXIXd) handelt

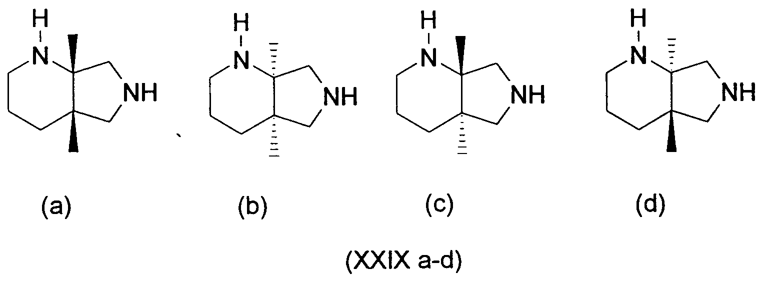

9. Verfahren gemäß Ansprüchen 1 bis 8, dadurch gekennzeichnet, daß zur Cyclisierung der Verbindungen der Formel (XIII) Kaliumcarbonat als Hilfsbase eingesetzt wird.

## Claims

1. One-pot process for the preparation of 3-quinolone carboxylic acid derivatives of the general formula (Ia) 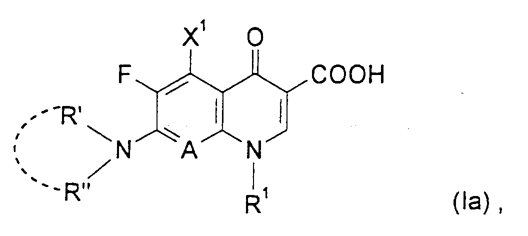 in which
R' and R'', together with the nitrogen atom to which they are bonded, form a monocyclic or bicyclic heterocycle which, where appropriate, can contain further nitrogen, oxygen or sulphur hetero atoms in all ring parts, and which, where appropriate can be substituted, and
in which
A represents CH, CF, CCl, C-OCH₃ or C-CH₃,
X¹ represents H, halogen, NH₂ or CH₃,
R¹ represents C₁-C₃-alkyl, FCH₂CH₂- or cyclopropyl, or phenyl or cyclopropyl which are optionally substituted once to three times by halogen,
characterized in that, without isolation of the intermediates, acid halides of the formula (XXVI) 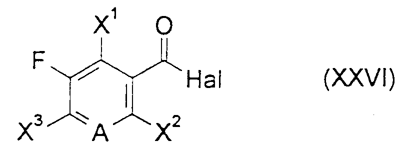 in which
Hal, X² and X³ represent fluorine or chlorine and A and X¹ have the abovementioned meanings, are either reacted in a solvent with a dimethylaminoacrylic acid ester of the formula (CH₃)₂N-CH=CH-COOR, after which the aminoacrylic ester (XIII) is produced by adding an amine R¹-NH₂, with amine exchange in the acrylic ester moiety of the primary product, or the acid halide is immediately reacted with an aminoacrylic ester of the formula R¹NH-CH=CH-COOR, in which case the above-described amine exchange is dispensed with and the compound (XIII) is produced directly, 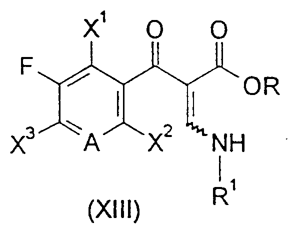 in which A, X¹ and R¹ have the meanings given for formula (Ia), X² and X³ represent halogen, and R represents a customary radical which is suitable for ester formation, preferably methyl, ethyl or propyl, which compound (XIII) is heated in a solvent with an auxiliary base, and thereby cyclized to form compounds of the general formula (XIV), 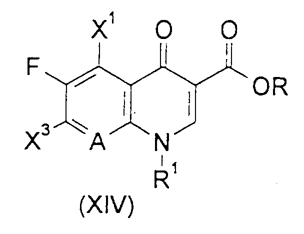 in which A, R, R¹ and X³ have the abovementioned meanings,
which are converted, without intermediate isolation, by reaction with heterocyclic compounds 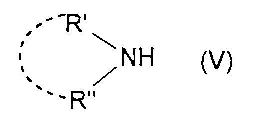 in which
R' and R'' have the abovementioned meanings,
into eaters of the general formula 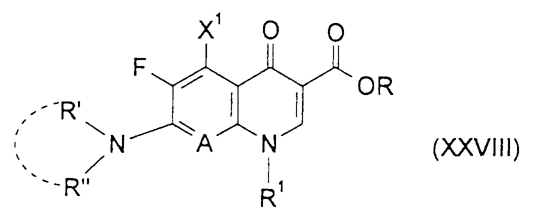 in which A, R, R¹, R', R'' and X¹ have the above-mentioned meanings, from which the 3-quinolonecarboxylic acid derivatives of the formula (Ia) result, by means of alkaline hydrolysis of the ester function, which derivatives are precipitated by neutralizing the reaction mixture.

2. One-pot process according to Claim 1, for the preparation of 3-quinolonecarboxylic acid derivatives of the general formula (Ib), 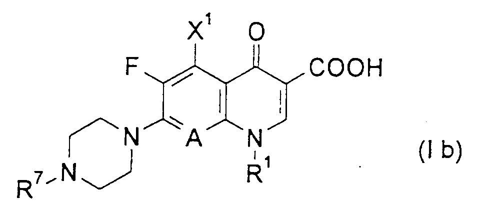 in which
A represents CH, CF, CCl, C-OCH₃ or C-CH₃,
X¹ represents H, halogen, NH₂ or CH₃,
R¹ represents C₁-C₃-alkyl, FCH₂CH₂- or cyclopropyl, or phenyl or cyclopropyl which are optionally substituted once to three times by halogen, and
R⁷ represents hydrogen, optionally substituted alkyl or phenyl, or a customary group which is suitable for protecting a nitrogen atom, preferably, however, ethyl,
characterized in that, without isolation of the intermediates, an acid halide of the formula (XXVI) 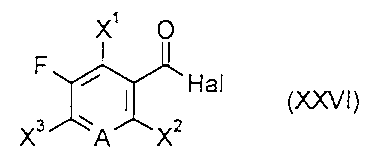 in which
Hal, X² and X³ represent fluorine or chlorine and A and X¹ have the meanings according to Claim 1, is reacted in a solvent with a dimethylaminoacrylic acid ester of the formula
(CH₃)₂N-CH=CH-COOR
after which the aminoacrylic ester (XIII), 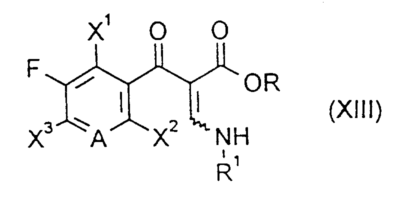 in which A, X¹ and R¹ have the meanings given for the formula (Ib), X² and X³ represent halogen, and R represents a customary organic radical which is suitable for ester formation, preferably methyl, ethyl or propyl, is produced by adding an amine R¹-NH₂, with amine exchange in the acrylic ester moiety of the primary product, and is then heated in a solvent containing an auxiliary base, and thereby cyclized to form compounds of the general formula (XIV), 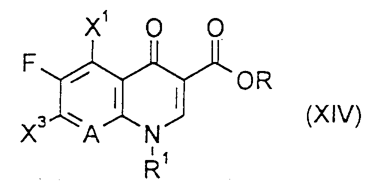 in which A, R, R¹, X¹ and X³ have the abovementioned meanings,
which are converted, by reaction with compounds of the general formula (XV) 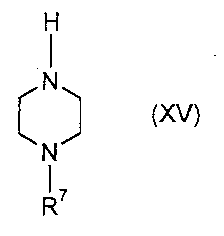 in which
R⁷ has the abovementioned meaning,
into esters of the general formula (XXVII) 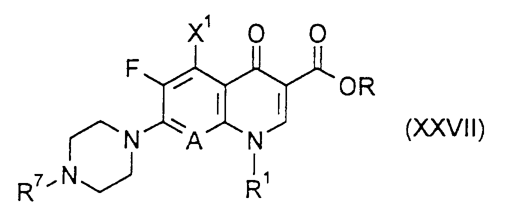 in which A, R, R¹, R⁷ and X¹ have the abovementioned meanings, from which the 3-quinolonecarboxylic acid derivatives of the formula (Ib) result, by means of alkaline hydrolysis of the ester function, and are precipitated by neutralizing the reaction mixture.

3. One-pot process according to Claim 1 for the preparation of 3-quinolonecarboxylic acid derivatives of the general formula (I) 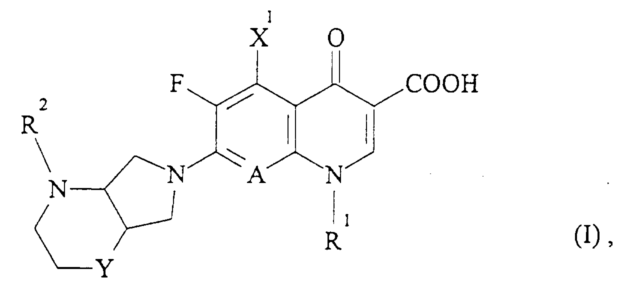 in which
A represents CH, CF, CCl, C-OCH₃ or C-CH₃,
X¹ represents H, halogen, NH₂ or CH₃,
Y represents CH₂ or O,
R¹ represents C₁-C₃-alkyl, FCH₂CH₂- or cyclopropyl, or phenyl or cyclopropyl which are optionally substituted once to three times by halogen,
R² represents hydrogen, 5-methyl-2-oxo-1,3-dioxolen-4-yl-methyl, C₂-C₅-oxoalkyl, CH₂-CO-C₆H₅, CH₂CH₂CO₂R⁶, R⁶O₂C-CH=C-CO₂R⁶, -CH=CH-CO₂R⁶ or CH₂CH₂-CN,
in which
R⁶ denotes hydrogen or C₁-C₃-alkyl,
characterized in that, without isolation of the intermediates, an acid halide of the formula 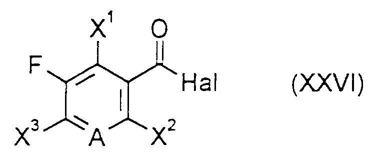 in which
Hal, X² and X³ represent fluorine or chlorine and A and X¹ have the meanings given for formula (I), is reacted in a solvent with a dimethylaminoacrylic acid ester of the formula (CH₃)₂N-CH=CH-COOR, after which the aminoacrylic ester (XIII), 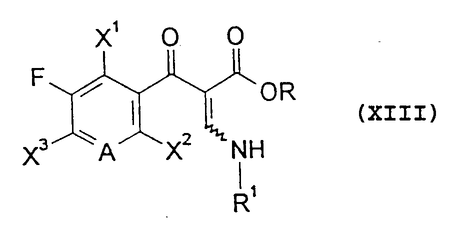 in which A, X¹ and R¹ have the meanings given for formula (I), X² and X³ represent halogen, and R represents a customary organic radical which is suitable for ester formation, preferably methyl, ethyl or phenyl, is produced by adding an amine R¹-NH₂, with amine exchange in the acrylic ester moiety of the primary product, which aminoacrylic ester (XIII) is heated in a solvent together with an auxiliary base and thereby cyclized to form compounds of the general formula (XIV), 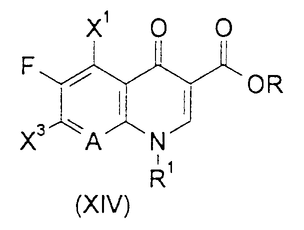 in which A, R, R¹, X¹ and X³ have the abovementioned meanings,
which are converted, by reaction with compounds of the general formula (XVII) 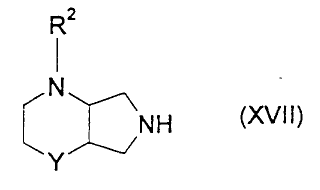 in which
R² represents hydrogen, 5-methyl-2-oxo-1,3-dioxolen-4-yl-methyl, C₂-C₅-oxoalkyl, CH₂-CO-C₆H₅, CH₂CH₂CO₂R⁶, R⁶O₂C-CH=C-CO₂R⁶, -CH=CH-CO₂R⁶ or CH₂CH₂-CN,
Y represents CH₂ or O,
into esters of the general formula (XVI) 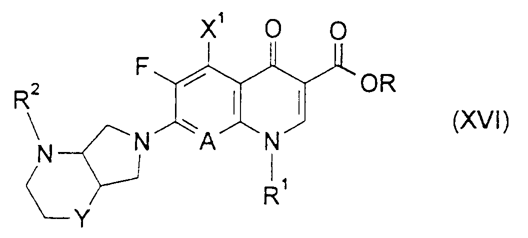 in which A, R, R¹, R², Y and X¹ have the above-mentioned meanings, from which the 3-quinolonecarboxylic acid derivatives of the formula (I) result, by means of alkaline hydrolysis of the ester function, which derivatives are precipitated by neutralizing the reaction mixture.

4. Process according to Claim 3, characterized in that acrylic esters of the formula (XIII) are employed in which
A represents CH, CF, CCl, C-OCH₃ or C-CH₃,
R represents a customary organic radical which is suitable for ester formation,
R¹ represents C₁-C₃-alkyl, FCH₂CH₂- or cyclopropyl, or phenyl or cyclopropyl which are optionally substituted once to three times by halogen,
X¹ represents H, halogen, NH₂ or CH₃, and
X² and X³ represent halogen,
and those compounds of the formula (XVII) in which
R² represents H, CH₂-O-CH₃, CH₂-O-C₆H₅, CH₂CH₂-CO-CH₃, CH₂CH₂CO₂R⁶, R⁶O₂C-CH=C-CO₂R⁶, -CH=CO₂R⁶ or CH₂CH₂CN,
in which
R⁶ denotes C₁-C₃-alkyl, and
Y represents CH₂ or O.

5. Process according to Claim 2, characterized in that those acid halides of the formula (XXVI) are employed in which
A represents CH,
X¹ represents hydrogen,
X² represents fluorine or chlorine, and
X³ represents fluorine,
and those compounds of the formula (XV) in which
R⁷ represents hydrogen or ethyl.

6. Process according to Claim 4, characterized in that acrylic eaters of the formula (XIII) are employed in which
A represents CCl or CF,
R represents CH₃ or C₂H₅,
R¹ represents cyclopropyl,
X¹ represents hydrogen,
X² represents fluorine or chlorine, and
X³ represents fluorine,
and those compounds (XVII) in which
R² represents hydrogen, and
Y represents CH₂.

7. Process according to Claims 1, 3, 4 and 6, characterized in that the compounds of the formula (XVII) are enantiomerically pure substances.

8. Process according to Claim 6, characterized in that the compound of the formula (XVII) is an enantiomerically pure compound from the group of the compounds of the formulae (XXIXa) to (XXIXd)

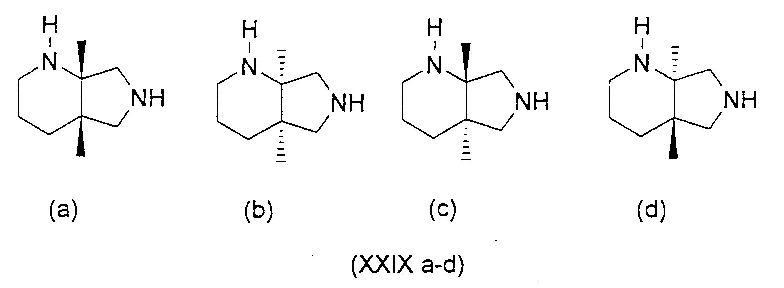

9. Process according to Claims 1 to 8, characterized in that potassium carbonate is employed as auxiliary base for cyclizing the compounds of the formula (XIII).

## Revendications

1. Procédé de production en réacteur unique de dérivés d'acide 3-quinolonecarboxylique de formule générale (Ia) 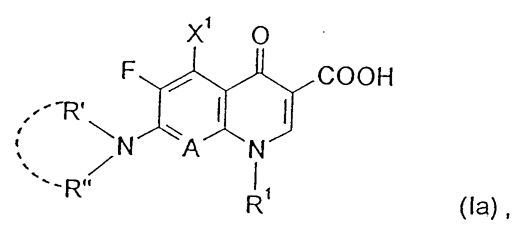 dans laquelle
R' et R'' forment, conjointement avec l'atome d'azote auquel ils sont liés, un hétérocycle monocyclique ou bicyclique qui peut contenir le cas échéant dans toutes parties cycliques d'autres hétéroatomes d'azote, d'oxygène ou de soufre et qui peut éventuellement être substitué, et
dans laquelle
A représente un groupe CH, DF, CCl, C-OCH₃, C-CH₃,
X¹ représente H, un halogène, un groupe NH₂, CH₃,
R¹ est un groupe alkyle en C₁ à C₃, FCH₂CH₂-, cyclopropyle, un groupe phényle ou cyclopropyle portant le cas échéant 1 à 3 substituants halogéno,
caractérisé en ce que, sans isolement des stades intermédiaires, un halogénure d'acide de formule (XXVI) 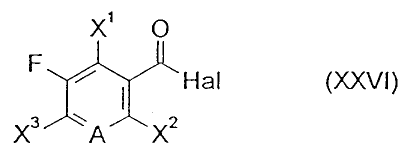 dans laquelle
Hal, X² et X³ représentent le fluor ou le chlore et A et X¹ ont la définition indiquée ci-dessus, est amené à réagir avec un ester d'acide diméthylaminoacrylique de formule (CH₃)₂N-CH=CH-COOR dans un solvant, après quoi l'ester aminoacrylique (XIII) est produit par addition d'une amine R¹-NH₂ avec échange d'amine dans la partie ester acrylique du produit primaire, ou bien l'halogénure d'acide est amené à réagir en même temps qu'avec un ester aminoacrylique de formule R¹NH-CH=CH-COOR, auquel cas l'échange d'amine décrit ci-dessus disparaît et le produit (XIII) 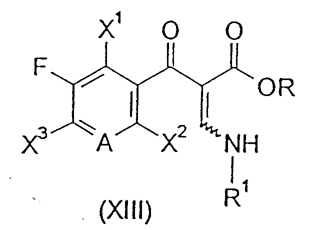 dans lequel A, X¹ et R¹ ont la définition indiquée pour la formule (La), X² et X³ représentent un halogène et R est un reste classique convenant pour la formation d'un ester, de préférence un reste méthyle, éthyle ou propyle, est immédiatement produit, il est chauffé dans un solvant avec une base auxiliaire et il est alors cyclisé en composés de formule générale (XIV) 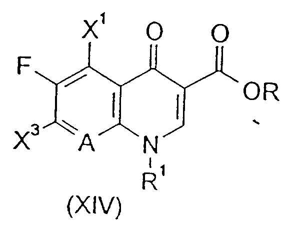 dans laquelle A, R, R¹ et X³ ont la définition indiquée ci-dessus,
qui sont transformés sans isolement intermédiaire par réaction avec des composés hétérocycliques 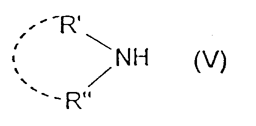 où
R' et R'' ont la définition indiquée ci-dessus
en esters de formule générale 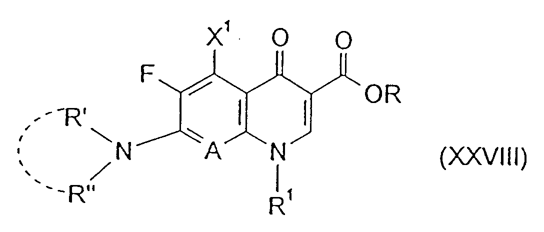 dans laquelle A, R, R¹, R', R'' et X¹ ont la définition indiquée ci-dessus, à partir desquels sont produits, par saponification alcaline de la fonction ester, les dérivés d'acide 3-quinolonecarboxyliques de formule (Ia), qui sont précipités par neutralisation du mélange réactionnel.

2. Procédé en réacteur unique suivant la revendication 1, pour la production de dérivés d'acide 3-quinolone-carboxyliques de formule générale (Ib), 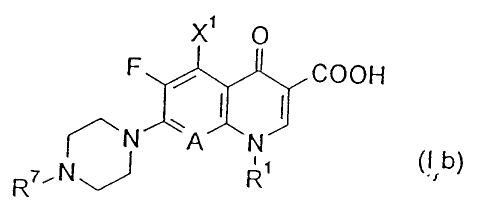 dans laquelle
A représente un groupe CH, CF, CCl, C-OCH₃, C-CH₃,
X¹ représente H, un halogène, un groupe NH₂, CH₃,
R¹ est un groupe alkyle en C₁ à C₃, FCH₂CH₂-, cyclopropyle, un groupe phényle ou cyclopropyle portant le cas échéant un à trois substituants halogéno et
R⁷ représente de l'hydrogène, un groupe alkyle ou phényle éventuellement substitué ou un groupe classique convenant pour la protection d'un atome d'azote, mais de préférence le groupe éthyle,
caractérisé en ce que, sans isolement des stades intermédiaires, un halogénure d'acide de formule (XXVI) 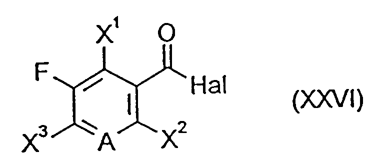 dans laquelle
Hal, X² et X³ représentent du fluor ou du chlore et A et X¹ ont la définition suivant la revendication 1, est amené à réagir avec un ester d'acide diméthylaminoacrylique de formule
(CH₃)₂N-CH=CH-COOR
dans un solvant, après quoi par addition d'une amine R¹-NH₂, l'ester aminoacrylique (XIII), est produit avec échange d'amine dans la partie ester acrylique du produit primaire 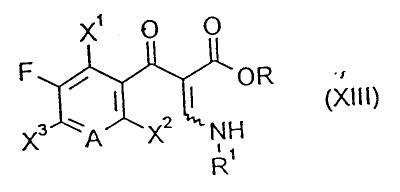 dans lequel A, X¹ et R¹ ont la définition indiquée pour la formule (Ib), X² et X³ représentent un halogène et R est un reste organique classique convenant pour la formation d'un ester, de préférence le reste méthyle, éthyle ou propyle, est chauffé dans un solvant avec une base auxiliaire et cyclisé alors en composés de formule générale (XIV) 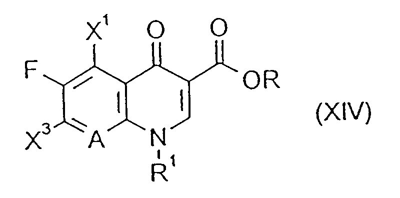 dans laquelle A, R, R¹, X¹ et X³ ont la définition indiquée ci-dessus,
qui sont transformés par réaction avec des composés de formule générale (XV) 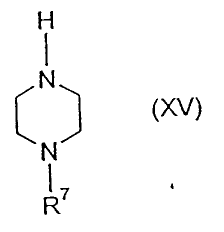 dans laquelle
R⁷ a la définition indiquée ci-dessus,
en esters de formule générale (XXVII) 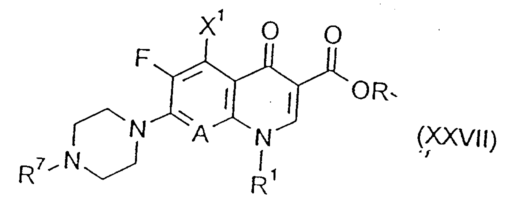 dans laquelle A, R, R¹, R⁷ et X¹ ont la définition indiquée ci-dessus, à partir desquels sont produits, par saponification alcaline de la fonction ester, les dérivés d'acide 3-quinolone-carboxylique de formule (Ib) qui sont précipités par neutralisation du mélange réactionnel.

3. Procédé en réacteur unique suivant la revendication 1 pour la production de dérivés d'acide 3-quinolone-carboxylique de formule générale (I) 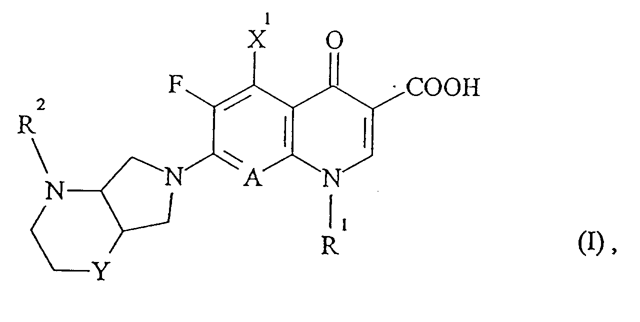 dans laquelle
A est un groupe CH, CF, CCl, C-OCH₃, C-CH₃,
X¹ représente H, un halogène, un groupe NH₂, CH₃,
Y représente CH₂ ou O,
R¹ est un groupe alkyle en C₁ à C₃, FCH₂CH₂-, cyclopropyle, un groupe phényle ou cyclopropyle portant le cas échéant un à trois substituants halogéno,
R² est de l'hydrogène, un groupe 5-méthyl-2-oxo-1,3-dioxolén-4-yl-méthyle, oxo-alkyle en C₂-C₅-, CH₂-CO-C₆H₅, CH₂CH₂CO₂R⁶, R⁶O₂C-CH=C-CO₂R⁶, -CH=CH-CO₂R⁶ ou CH₂CH₂-CN,
où
R⁶ est de l'hydrogène ou un alkyle en C₁ à C₃,
caractérisé en ce que, sans isolement des stades intermédiaires, un halogénure d'acide de formule 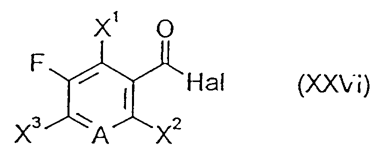 dans laquelle
Hal, X² et X³ représentent du fluor ou du chlore et A et X¹ ont la définition indiquée pour la formule (I), est amené à réagir avec un ester d'acide diméthylaminoacrylique de formule (CH₃)₂N-CH=CH-COOR dans un solvant, après quoi, par addition d'une amine R¹NH₂, l'ester aminoacrylique (XIII) est produit avec échange d'amine dans la partie ester acrylique du produit primaire 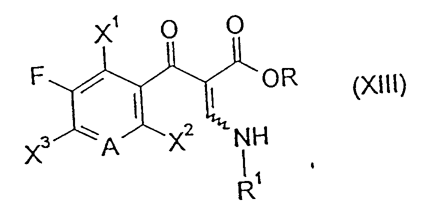 dans lequel A, X¹ et R¹ ont la définition indiquée pour la formule (I), X² et X³ représentent un halogène et R est un reste organique classique convenant pour la formation d'un ester, de préférence un reste méthyle, éthyle ou phényle, est chauffé dans un solvant avec une base auxiliaire et cyclisé alors en composés de formule générale (IV) 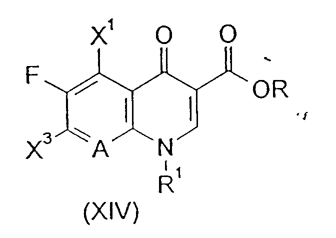 dans laquelle A, R, R¹, X¹ et X³ ont la définition indiquée ci-dessus,
qui sont transformés par réaction avec des composés de formule générale (XVII) 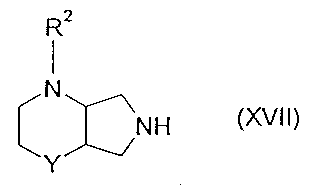 dans laquelle
R² représente de l'hydrogène, un groupe 5-méthyl-2-oxo-1,3-dioxolén-4-yl-méthyle, oxo-alkyle en C₂-C₅-, CH₂-CO-C₆H₅, CH₂CH₂CO₂R⁶, R⁶O₂C-CH=C-CO₂R⁶, -CH=CH-CO₂R⁶ ou CH₂CH₂-CN,
Y représente CH₂ ou O,
en esters de formule générale (XVI) 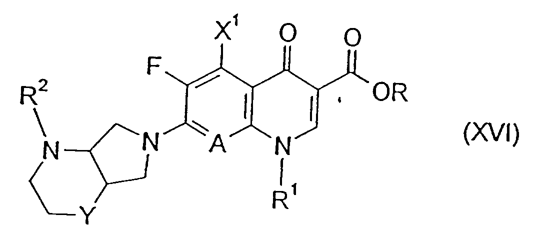 dans laquelle A, R, R¹, R², Y et X¹ ont la définition indiquée ci-dessus, à partir desquels sont produits par saponification alcaline de la fonction ester les dérivés d'acide quinolone-carboxylique de formule (I) qui sont précipités par neutralisation du mélange réactionnel.

4. Procédé suivant la revendication 3, caractérisé en ce qu'on utilise des esters acryliques de formule (XIII) dans lesquels
A représente CH, CF, CCl, C-OCH₃, C-CH₃,
R est un reste organique classique convenant pour la formation d'un ester,
R¹ est un groupe alkyle en C₁ à C₃, FCH₂CH₂-, cyclopropyle, un groupe phényle ou cyclopropyle portant le cas échéant un à trois substituants halogéno,
X¹ représente H, un halogène, un groupe NH₂, CH₃ et
X² et X³ représentent un halogène
et comme composés de formule (XVII) des composés dans lesquels,
R² représente de l'hydrogène, CH₂6O-CH₃, CH₂-O-C₆H₅, CH₂CH₂-CO-CH₃, CH₂CH₂CO₂R⁶, R⁶O₂C-CH=C-CO₂R⁶, -CH=CO₂R⁶, CH₂CH₂CN où
R⁶ est un groupe alkyle en C₁ à C₃ et
Y représente CH₂ ou O.

5. Procédé suivant la revendication 2, caractérisé en ce qu'on utilise comme halogénures d'acides de formule (XXVI) des halogénures dans lesquels
A représente CH,
X¹ est de l'hydrogène,
X² est du fluor ou du chlore et
X³ est du fluor,
et comme composés de formule (XV), des composés dans lesquels
R⁷ est de l'hydrogène ou un groupe éthyle.

6. Procédé suivant la revendication 4, caractérisé en ce qu'on utilise des esters acryliques de formule (XIII) dans lesquels
A représente CCl ou CF
R est un groupe CH₃ ou C₂H₅
R¹ est un groupe cyclopropyle
X¹ est de l'hydrogène
X² est du fluor ou du chlore et
X³ est du fluor
et comme composés (XVII) des composés dans lesquels
R² est de l'hydrogène et
Y est un groupe CH₂.

7. Procédé suivant les revendications 1, 3, 4 et 6, caractérisé en ce que les composés de formule (XVII) sont des substances énantiomères pures.

8. Procédé suivant la revendication 6, caractérisé en ce que le composé de formule (XVII) est un composé énantiomère pur du groupe des composés de formules (XXIXa) à (XXIXd).

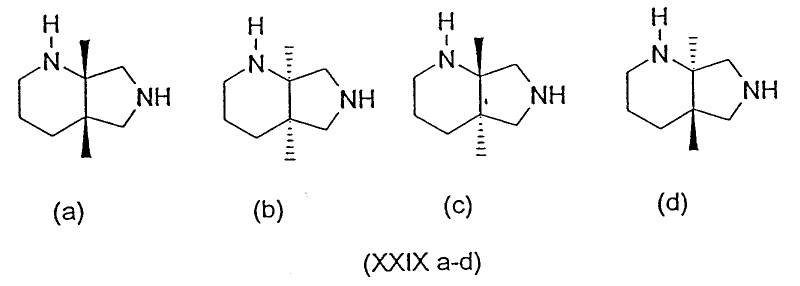

9. Procédé suivant les revendications 1 à 8, caractérisé en ce qu'on utilise comme base auxiliaire le carbonate de potassium pour la cyclisation des composés de formule (XIII).
